# EUROPEAN PATENT APPLICATION

(11) **EP 3 758 007 A1**
(43) Date of publication of application: **30.12.2020**
(21) Application number: 18910401.1
(22) Date of filing: 25.12.2018
(51) Int. Cl.: G16H 10/60, A61B 5/00, G16H 20/60

(54) **HEALTH MANAGEMENT ASSISTANCE SYSTEM**

(30) Priority: 23.03.2018 JP 2018056674
(71) Applicant: Aichi Health Promotion Public Interest Foundation, Higashiura-cho Chita-gun Aichi 4702101 (JP)
(72) Inventor: TSUSHITA Kazuyo, Chita-gun Aichi 4702101 (JP)
(74) Representative: TBK
(86) International application number: PCT/JP2018/047562
(87) International publication number: WO 2019/181129

(57) **Abstract**

Health guidance according to a user's situation is provided while the burden on a healthcare leader is reduced, in supporting the healthcare for the user.

A healthcare support system that supports healthcare for a user includes a terminal computer and a management server. The management server includes an acquired information database unit that accumulates biological information and behavior information transmitted from the terminal computer; an evaluation standard setting unit that sets an evaluation standard for each of a plurality of management items related to the healthcare of the user; an evaluation selector that selects an evaluation message for each of the management items based on the biological information, the behavior information, and the evaluation standard; and a display instruction unit that instructs the terminal computer to display the evaluation message for each of the management items. The evaluation standard setting unit sets an evaluation standard related to at least one of the management items based on at least either the biological information or the behavior information accumulated in the acquired information database unit.

## Description

### TECHNICAL FIELD

The present disclosure relates to a healthcare support system.

### BACKGROUND ART

To prevent and treat lifestyle-related diseases, it is important to improve lifestyle. However, it is not easy to change the lifestyle that has continued for many years. To address this problem, various systems to support healthcare for users have been suggested.

Patent Document 1 discloses a system that connects a patient terminal device and a healthcare worker terminal device together via a management server. The system of Patent Document 1 transmits measurement data of a patient from the patient terminal device to the healthcare worker terminal device via the management server, and transmits a healthcare worker's advice from the healthcare worker terminal device to the patient terminal device via the management server.

Patent Document 2 discloses a system that connects a mobile phone and a server together via the Internet. In the system of Patent Document 2, the mobile phone collects measurement data from a health measurement device via infrared communication, transmits the measurement data to the server via the Internet, and displays the measurement data accumulated in the server in an optional display format.

### CITATION LIST

### PATENT DOCUMENTS

PATENT DOCUMENT 1: Japanese Unexamined Patent Publication No. 2011-180817
PATENT DOCUMENT 2: Japanese Unexamined Patent Publication No. 2006-99301

### SUMMARY OF THE INVENTION

### TECHNICAL PROBLEM

No adequate consideration has been given to providing health guidance according to a user's situation while lessening the burden on a healthcare leader in supporting healthcare for the user.

### SOLUTION TO THE PROBLEM

(1) A healthcare support system according to an aspect of the present disclosure supports healthcare of a user. The healthcare support system includes: a terminal computer operable by the user; and a management server capable of exchanging data with the terminal computer via a computer network. The terminal computer includes: a biological information acquisition unit configured to acquire biological information of the user from a biological information detection device via short-range wireless communication, the biological information detection device detecting the biological information; a behavior information acquisition unit configured to acquire behavior information indicating a behavior of the user based on input manipulated by the user; and a terminal information transmitter configured to transmit the biological information acquired by the biological information acquisition unit and the behavior information acquired by the behavior information acquisition unit to the management server. The management server includes: an acquired information database unit configured to accumulate the biological information and the behavior information transmitted from the terminal computer; an evaluation standard setting unit configured to set an evaluation standard for each of a plurality of management items related to the healthcare of the user; an evaluation selector configured to select an evaluation message for evaluating efforts for the healthcare of the user during a predetermined period for each of the management items based on the biological information and the behavior information acquired during the predetermined period and accumulated in the acquired information database unit and the evaluation standard for each of the management items; and a display instruction unit configured to instruct the terminal computer to display the evaluation message for each of the management items such that the user is able to recognize the evaluation message for each of the management items. According to the healthcare support system of this aspect, based on the biological information and the behavior information acquired during the predetermined period, the management server selects the evaluation message for evaluating the user's healthcare efforts for each of the management items to allow the user to recognize these evaluation messages in the terminal computer. Consequently, it is possible to implement health guidance according to the user's situation while reducing the burden on a healthcare leader.
(2) In the aforementioned healthcare support system, based on the biological information and the behavior information acquired during the predetermined period and accumulated in the acquired information database unit and the evaluation standard for each of the management items, the evaluation selector of the management server may select the evaluation message for each of the management items, and selects a character image corresponding to the evaluation message for each of the management items. The display instruction unit of the management server may instruct the terminal computer to display the evaluation message and the character image for each of the management items such that the user is able to recognize the evaluation message and the character image for each of the management items. According to the healthcare support system of this embodiment, evaluating the user's healthcare efforts using the character image in addition to the evaluation message can improve the user's eagerness to make healthcare efforts.
(3) In the aforementioned healthcare support system, the biological information detection device, which provides the biological information acquisition unit with the biological information, may include at least one of a body composition meter, a blood pressure meter, and an activity meter. According to the healthcare support system of this aspect, the user's healthcare efforts can be evaluated based on the biological information detectable by at least one of the body composition meter, the blood pressure meter, and the activity meter.
(4) In the aforementioned healthcare support system, the behavior information acquired by the behavior information acquisition unit may include information indicating a dietary behavior of the user. According to the healthcare support system of this aspect, the user's healthcare efforts can be evaluated based on the behavior information indicating the dietary behavior of the user.
(5) In the aforementioned healthcare support system, the management items may comprise at least two of: a first management item to manage a recording frequency with which the biological information and the behavior information have been recorded; a second management item to manage the number of steps of the user; a third management item to manage a physical activity amount of the user; a fourth management item to manage dietary life of the user; a fifth management item to manage a weight of the user; a sixth management item to manage a blood pressure of the user; and a seventh management item to comprehensively manage the other ones of the management items. According to the healthcare support system of this aspect, efforts for prevention and treatment of type II diabetes can be evaluated as the user's healthcare efforts.
(6) In the aforementioned healthcare support system, the evaluation standard setting unit of the management server may set the evaluation standard related to at least one of the management items based on at least either biological information or behavior information accumulated in the acquired information database unit. According to the healthcare support system of this aspect, the past performance for user's healthcare can be reflected in the evaluation standard.
   In the aforementioned healthcare support system, the evaluation standard setting unit of the management server may set the evaluation standard related to at least one of the management items based on at least either biological information or behavior information different from biological information and behavior information evaluated in the at least one of the management items, out of the biological information and the behavior information accumulated in the acquired information database unit. According to the healthcare support system of this aspect, the past performance for user's healthcare can be reflected in the evaluation standard.
(7) A management server according to an aspect of the present disclosure is capable of exchanging data with a terminal computer operable by a user via a computer network in a healthcare support system that supports healthcare of the user. The management server includes: an information acquisition unit configured to acquire biological information of the user and behavior information indicating a behavior of the user from the terminal computer via the computer network, the biological information being acquired by the terminal computer from a biological information detection device via short-range wireless communication, the biological information detection device detecting the biological information, the behavior information being acquired by the terminal computer based on input manipulated by the user; an acquired information database unit configured to accumulate the biological information and the behavior information acquired by the information acquisition unit; an evaluation standard setting unit configured to set an evaluation standard for each of a plurality of management items related to the healthcare of the user; an evaluation selector configured to select an evaluation message for evaluating efforts for the healthcare of the user during a predetermined period for each of the management items based on the biological information and the behavior information acquired during the predetermined period and accumulated in the acquired information database unit and the evaluation standard for each of the management items; and a display instruction unit configured to instruct the terminal computer to display the evaluation message for each of the management items such that the user is able to recognize the evaluation message for each of the management items. According to the terminal computer of this embodiment, the management server can select the evaluation message for evaluating the user's healthcare efforts for each of the management items, based on the biological information and the behavior information acquired during the predetermined period, to allow the user to recognize these evaluation messages in the terminal computer. Consequently, it is possible to implement health guidance according to a user's situation while reducing the burden on a healthcare leader.

The present disclosure can be implemented in various forms in addition to the healthcare support system and the management server. The present disclosure can be implemented in any form, such as the aforementioned terminal computer program, the aforementioned management server program, a method for supporting healthcare of a user.

### BRIEF DESCRIPTION OF THE DRAWINGS

[FIG. 1] FIG. 1 is a block diagram showing a healthcare support system.
[FIG. 2] FIG. 2 is a block diagram showing a detailed configuration of a terminal computer.
[FIG. 3] FIG. 3 illustrates an example of a screen for acquiring dietary information from a user.
[FIG. 4] FIG. 4 illustrates an example of a screen for displaying evaluation messages and character images.
[FIG. 5] FIG. 5 is a block diagram showing a detailed configuration of a management server.
[FIG. 6] FIG. 6 illustrates exemplary evaluation message data related to a first evaluation item "Recording Frequency."
[FIG. 7] FIG. 7 illustrates exemplary evaluation message data related to a second evaluation item "Number of Steps."
[FIG. 8] FIG. 8 illustrates exemplary evaluation message data related to a third evaluation item "Physical Activity Amount."
[FIG. 9] FIG. 9 illustrates exemplary evaluation message data related to a fourth evaluation item "Dietary Life."
[FIG. 10] FIG. 10 illustrates exemplary evaluation message data related to a fifth evaluation item "Weight."
[FIG. 11] FIG. 11 illustrates exemplary evaluation message data related to a sixth evaluation item "Blood Pressure."
[FIG. 12] FIG. 12 illustrates exemplary evaluation message data related to a seventh evaluation item "Comprehensive Evaluation."
[FIG. 13] FIG. 13 illustrates operation of the healthcare support system.
[FIG. 14] FIG. 14 is a flowchart showing an information collecting process performed by a terminal computer.
[FIG. 15] FIG. 15 is a flowchart showing an information accumulating process performed by a management server.
[FIG. 16] FIG. 16 illustrates operation of the healthcare support system.

### DESCRIPTION OF EMBODIMENTS

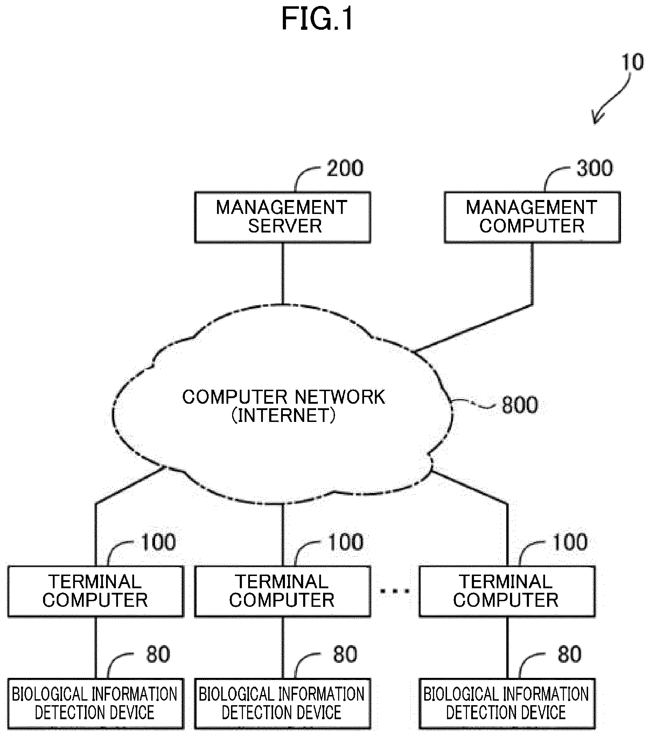

FIG. 1 is a block diagram showing a healthcare support system 10. The healthcare support system 10 supports healthcare for users. The healthcare support system 10 includes terminal computers 100 and a management server 200. The healthcare support system 10 further includes a management computer 300.

The terminal computers 100 of the healthcare support system 10 are each operable by a user. The terminal computers 100 may be either smart phones or tablets. The terminal computers 100 are respectively capable of communicating with biological information detection devices 80 via short-range wireless communication.

The biological information detection devices 80 each detect biological information of a user who uses an associated one of the terminal computers 100. The biological information detection devices 80 have a communication function conforming to the Bluetooth (registered trademark) standard as a short-range wireless communication function. The biological information detection devices 80 include a body composition meter, a blood pressure meter, and an activity meter. The body composition meter can measure the user's weight, the user's body fat percentage, the user's visceral fat level, the user's subcutaneous fat percentage, the user's basal metabolism, the user's skeletal muscle percentage, the user's body mass index (BMI), and other elements as the biological information of the user. The blood pressure meter can measure the user's systolic blood pressure, the user's diastolic blood pressure, and other blood pressures as the biological information of the user. The activity meter can measure the number of the user's steps, metabolic equivalents (METs) and other elements as the biological information of the user.

The management server 200 of the healthcare support system 10 is capable of exchanging data with the terminal computer 100 via a computer network 800. The management server 200 can exchange data with a plurality of the terminal computers 100 via the computer network 800. The management server 200 is a cloud server capable of exchanging data with the terminal computers 100 by cloud computing. The computer network 800 includes the Internet, a mobile phone communication network, and a wireless local area network.

The management computer 300 of the healthcare support system 10 is capable of exchanging data with the management server 200 via the computer network 800. The management computer 300 may be any one of a personal computer, a smart phone, or a tablet. The management computer 300 controls the management server 200 based on input manipulated by an administrator who manages the healthcare support system 10.

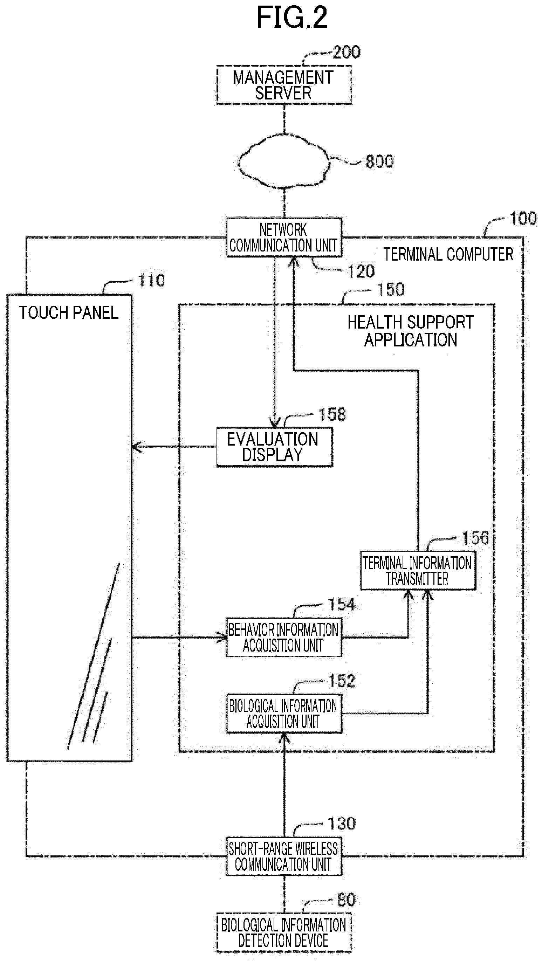

FIG. 2 is a block diagram showing a detailed configuration of one of the terminal computers 100. The terminal computer 100 includes a touch panel 110, a network communication unit 120, and a short-range wireless communication unit 130. The touch panel 110 senses input manipulated by a user, and displays an image. The network communication unit 120 is connectable to the computer network 800. The network communication unit 120 has the function of connecting to the computer network 800 through the mobile phone communication network and the function of connecting to the computer network 800 through the wireless local area network. The short-range wireless communication unit 130 is capable of communicating with the associated biological information detection device 80 via the short-range wireless communication. The short-range wireless communication unit 130 has a communication function conforming to the Bluetooth (registered trademark) standard.

The terminal computer 100 further includes a biological information acquisition unit 152, a behavior information acquisition unit 154, a terminal information transmitter 156, and an evaluation display 158 to support healthcare for the user. In the terminal computer 100, elements related to the health support function are implemented in the form of software based on a health support application 150 serving as application software. At least some of the elements related to the health support function may also be implemented in the form of hardware based on a circuit configuration.

The biological information acquisition unit 152 of the terminal computer 100 acquires biological information from the biological information detection device 80 via the short-range wireless communication. The biological information acquisition unit 152 acquires the biological information from the biological information detection device 80 through the short-range wireless communication unit 130. The biological information acquired by the biological information acquisition unit 152 includes the number of steps, metabolic equivalents, weight, systolic blood pressure, and diastolic blood pressure.

The behavior information acquisition unit 154 of the terminal computer 100 acquires behavior information indicating the behavior of the user based on input manipulated by the user. The behavior information acquisition unit 154 acquires the behavior information from the user through the touch panel 110. The behavior information acquired by the behavior information acquisition unit 154 includes dietary information indicating the user's behavior related to dietary life. The behavior information acquired by the behavior information acquisition unit 154 may indicate the user's behavior related to exercise or the user's behavior related to hospital visits.

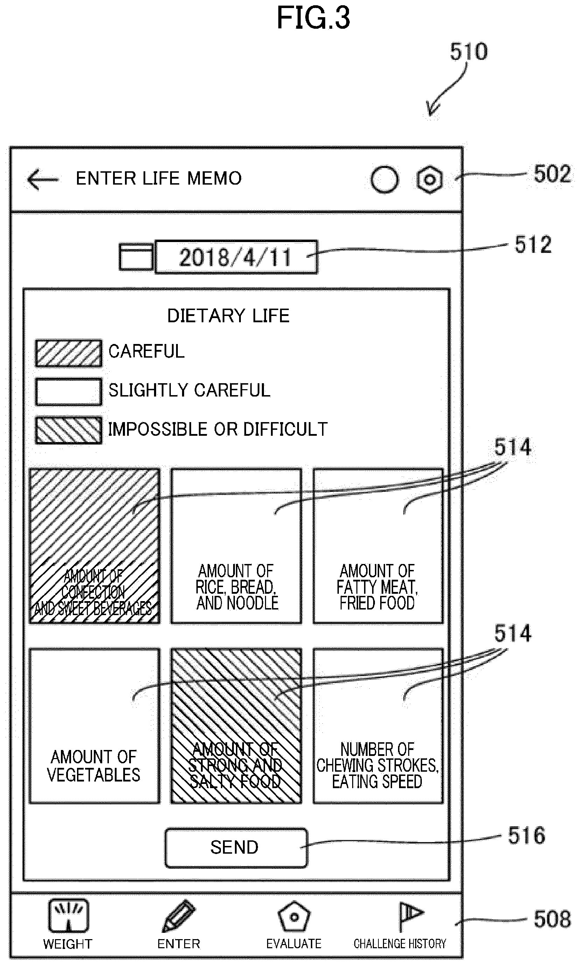

FIG. 3 illustrates an example of a screen 510 for acquiring dietary information from a user. The screen 510 is a graphical user interface (GUI) displayed on the touch panel 110 based on the function of the health support application 150. The screen 510 includes a title bar 502, a tab 508, a date input field 512, a plurality of input buttons 514, and a send button 516.

The title bar 502 of the screen 510 displays a character string indicating a title of the screen 510. The title bar 502 is positioned at an upper end of the screen 510. The title bar 502 may include a menu button to shift to the top page or a menu button to shift to a setting screen.

The tab 508 of the screen 510 includes various menu buttons. The tab 508 is positioned at a lower end of the screen 510. The tab 508 may include a menu button to shift to a screen that displays information on the user's weight. The tab 508 may include a menu button to shift to the screen 510 from another screen. The tab 508 may include a menu button to shift to a screen that displays how the user has used the health support application 150.

The date input field 512 of the screen 510 receives the input of a date on which dietary behaviors input through the input buttons 514 were performed from the user. The date input field 512 receives the input of the date from the user based on input manipulated by the user on the touch panel 110.

The input buttons 514 of the screen 510 receive the input of dietary information from the user. The input buttons 514 correspond to items related to different dietary behaviors. The color of each of the input buttons 514 changes in the order of green, yellow, and red every time the user performs an input operation on the touch panel 110. The green input button 514 receives information indicating that the user has been "careful" as the dietary information on the associated item. The yellow input button 514 receives information indicating that the user has been "slightly careful" as the dietary information on the associated item. The red input button 514 receives information indicating that it has been "impossible or difficult" for the user to be careful as the dietary information on the associated item.

The send button 516 of the screen 510 receives the input manipulated by the user to transmit the information entered into the date input field 512 and the input buttons 514 to the management server 200. When the send button 516 has received the manipulated input, the behavior information acquisition unit 154 acquires the date entered into the date input field 512 and the dietary information entered into the plurality of input buttons 514 as behavior information to be transmitted to the management server 200.

Returning to FIG. 2, the terminal information transmitter 156 of the terminal computer 100 transmits the biological information acquired by the biological information acquisition unit 152 and the behavior information acquired by the behavior information acquisition unit 154 to the management server 200. The terminal information transmitter 156 transmits the biological information and the behavior information to the management server 200 through the network communication unit 120.

The evaluation display 158 of the terminal computer 100 displays an evaluation message, which is selected by the management server 200 based on biological information and behavior information acquired during a predetermined period, in accordance with an instruction from the management server 200. The evaluation message is a sentence for evaluating the user's healthcare efforts during the predetermined period for each of a plurality of management items related to the user's healthcare. The evaluation display 158 displays a character image selected by the management server 200 in accordance with the evaluation message, in addition to the evaluation message.

In the present embodiment, the evaluation display 158 displays the evaluation message and the character image received from the management server 200 on the touch panel 110. In another embodiment, a database storing various evaluation messages and various character images may be prepared in advance in the health support application 150, and the evaluation display 158 may receive symbols indicating the evaluation messages and character images from the management server 200, read the evaluation messages and character images corresponding to these symbols from the database, and display the evaluation messages and character images on the touch panel 110.

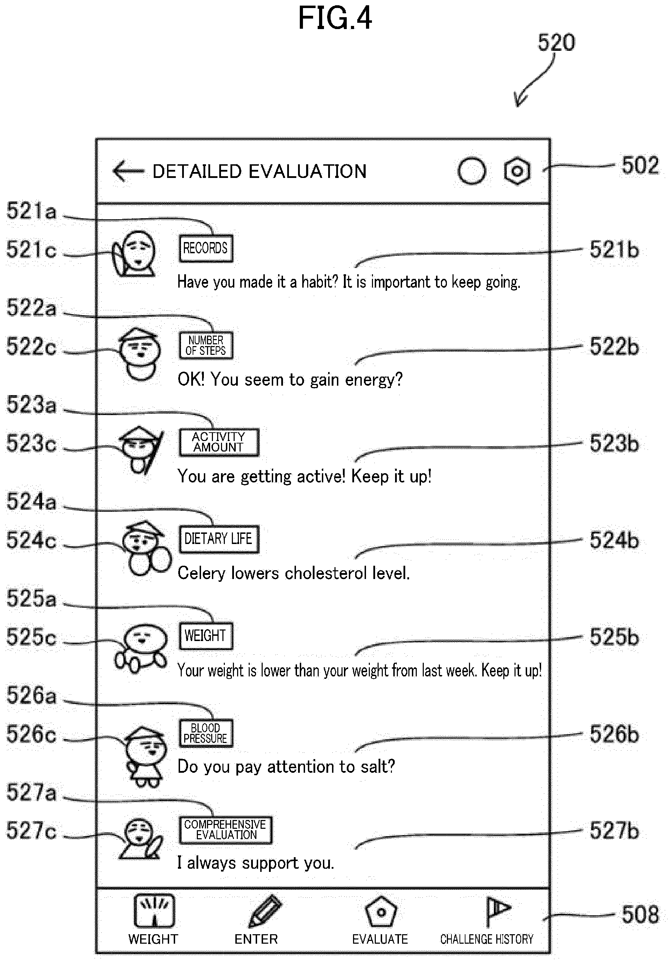

FIG. 4 illustrates an example of a screen 520 for displaying the evaluation messages and the character images. The screen 520 is a graphic user interface displayed on the touch panel 110 based on the function of the health support application 150. The screen 520 includes a title bar 502 and a tab 508 similarly to the screen 510. The screen 520 further includes display sections 521a to 521c related to a first management item, display sections 522a to 522c related to a second management item, display sections 523a to 523c related to a third management item, display sections 524a to 524c related to a fourth management item, display sections 525a to 525c related to a fifth management item, display sections 526a to 526c related to a sixth management item, and display sections 571a to 571c related to a seventh management item, in accordance with the plurality of management items related to the user's healthcare.

The first management item is provided to manage the recording frequency with which the biological information and the behavior information have been recorded. The display section 521a displays "RECORDS" as a character string indicating the first management item. The display section 521b displays an evaluation message selected by the management server 200 in relation to the first management item. The display section 521c displays a character image selected by the management server 200 in relation to the first management item.

The second management item is provided to manage the number of steps of the user. The display section 522a displays "NUMBER OF STEPS" as a character string indicating the second management item. The display section 522b displays an evaluation message selected by the management server 200 in relation to the second management item. The display section 522c displays a character image selected by the management server 200 in relation to the second management item.

The third management item is provided to manage the user's physical activity amount. The display section 523a displays "ACTIVITY AMOUNT" as a character string indicating the third management item. The display section 523b displays an evaluation message selected by the management server 200 in relation to the third management item. The display section 523c displays a character image selected by the management server 200 in relation to the third management item.

The fourth management item is provided to manage the user's dietary life. The display section 524a displays "DIETARY LIFE" as a character string indicating the fourth management item. The display section 524b displays an evaluation message selected by the management server 200 in relation to the fourth management item. The display section 524c displays a character image selected by the management server 200 in relation to the fourth management item.

The fifth management item is provided to manage the user's weight. The display section 525a displays "WEIGHT" as a character string indicating the fifth management item. The display section 525b displays an evaluation message selected by the management server 200 in relation to the fifth management item. The display section 525c displays a character image selected by the management server 200 in relation to the fifth management item.

The sixth management item is provided to manage the user's blood pressure. The display section 526a displays "BLOOD PRESSURE" as a character string indicating the sixth management item. The display section 526b displays an evaluation message selected by the management server 200 in relation to the sixth management item. The display section 526c displays a character image selected by the management server 200 in relation to the sixth management item.

The seventh management item is provided to comprehensively manage the other management items of the plurality of management items. The display section 527a displays "COMPREHENSIVE EVALUATION" as a character string indicating the seventh management item. The display section 527b displays an evaluation message selected by the management server 200 in relation to the seventh management item. The display section 527c displays a character image selected by the management server 200 in relation to the seventh management item.

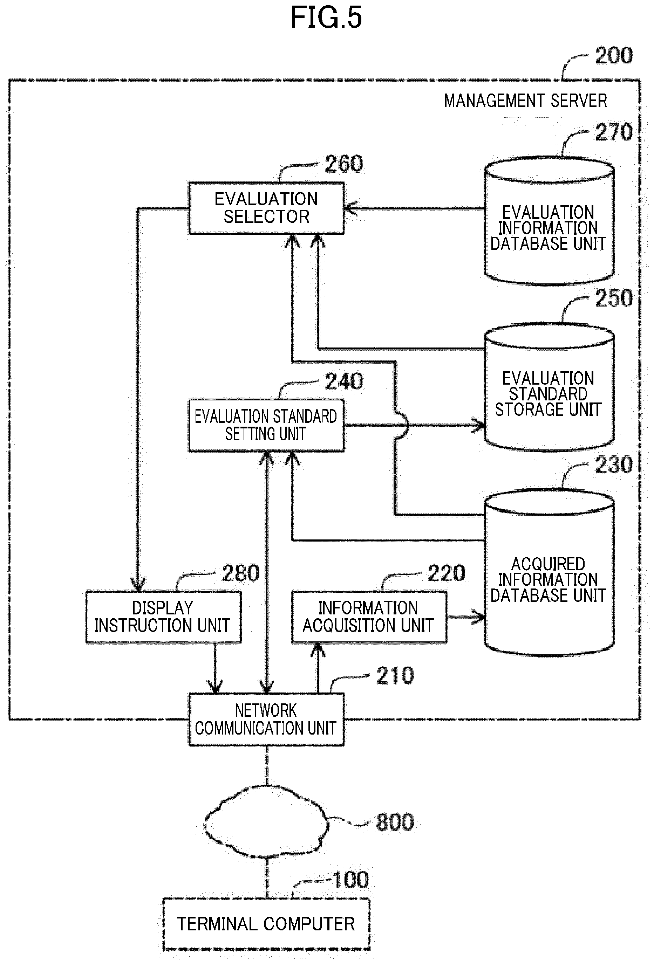

FIG. 5 is a block diagram showing a detailed configuration of the management server 200. The management server 200 includes a network communication unit 210. The network communication unit 210 is connectable to the computer network 800. The management server 200 further includes an information acquisition unit 220, an acquired information database unit 230, an evaluation standard setting unit 240, an evaluation standard storage unit 250, an evaluation selector 260, an evaluation information database unit 270, and a display instruction unit 280 as a health support function for supporting healthcare for users. In the management server 200, elements related to the health support function are implemented in the form of software based on a computer program. At least some of the elements in the management server 200 may also be implemented in the form of hardware based on a circuit configuration.

The information acquisition unit 220 of the management server 200 acquires the biological information and the behavior information transmitted from the terminal computer 100. The information acquisition unit 220 acquires the biological information and the behavior information through the network communication unit 210. The information acquisition unit 220 may directly receive at least part of the biological information and the behavior information from the terminal computer 100 via the computer network 800, and may indirectly receive at least part of the biological information and the behavior information from another database server that temporarily accumulates information from the terminal computer 100.

The acquired information database unit 230 of the management server 200 accumulates the biological information and the behavior information transmitted from the terminal computer 100. The acquired information database unit 230 accumulates biological information and behavior information for each user. The acquired information database unit 230 accumulates the biological information and the behavior information together with information indicating the date on which each piece of information has been recorded.

The evaluation standard setting unit 240 of the management server 200 sets an evaluation standard for each of the plurality of management items related to the user's healthcare. The evaluation standard setting unit 240 sets an evaluation standard for each of the first to seventh management items. The evaluation standard setting unit 240 can set an evaluation standard related to at least one of the plurality of management items as a fixed value. The evaluation standard setting unit 240 can set an evaluation standard related to at least one of the plurality of management items based on at least either the biological information or the behavior information accumulated in the acquired information database unit 230. The evaluation standard setting unit 240 may set an evaluation standard related to at least one of the plurality of management items based on an instruction from the management computer 300.

The management items, of which the evaluation standards are set by the evaluation standard setting unit 240, include the first management item "RECORDING FREQUENCY," the second management item "NUMBER OF STEPS," the third management item "PHYSICAL ACTIVITY AMOUNT," the fourth management item "DIETARY LIFE," the fifth management item "WEIGHT," the sixth management item "BLOOD PRESSURE," and the seventh management item "COMPREHENSIVE EVALUATION." The evaluation standard of the first management item "RECORDING FREQUENCY" is a fixed value. The evaluation standard of the second management item "NUMBER OF STEPS" is a fixed value. The evaluation standard of the third management item "PHYSICAL ACTIVITY AMOUNT" is a fixed value. The evaluation standard of the fourth management item "DIETARY LIFE" is a fixed value. The evaluation standard of the fifth management item "WEIGHT" is a variation value that is set based on the biological information accumulated in the acquired information database unit 230. The evaluation standard of the sixth management item "BLOOD PRESSURE" is a fixed value. The evaluation standard of the seventh management item "COMPREHENSIVE EVALUATION" is a fixed value.

The evaluation standard storage unit 250 of the management server 200 stores the evaluation standard set for each of the plurality of management items by the evaluation standard setting unit 240.

The evaluation selector 260 of the management server 200 selects an evaluation message for evaluating the user's healthcare efforts during a predetermined period (the preceding one week in the present embodiment) for each of the plurality of management items based on the biological information and the behavior information acquired during the predetermined period and accumulated in the acquired information database unit 230 and the evaluation standard stored in the evaluation standard storage unit 250 for each of the plurality of management items. The evaluation selector 260 selects a character image corresponding to the evaluation message for each of the plurality of management items, in addition to the evaluation message. The evaluation selector 260 selects an evaluation message and a character image stored in advance in the evaluation information database unit 270, based on the evaluation standards stored in the evaluation standard storage unit 250. The evaluation selector 260 selects the evaluation message on a regular basis (for example, twice a week).

The evaluation information database unit 270 of the management server 200 stores a plurality of evaluation messages and a plurality of character images in correlation with the evaluation standards stored in the evaluation standard storage unit 250. The evaluation information database unit 270 is capable of updating the evaluation messages and the character images based on an instruction from the management computer 300.

The display instruction unit 280 of the management server 200 instructs the terminal computer 100 to display the evaluation message for each of the plurality of management items selected by the evaluation selector 260 such that the user can recognize the evaluation message for each of the plurality of management items. The display instruction unit 280 instructs the terminal computer 100 to display the character image selected by the evaluation selector 260, in addition to the evaluation message. The display instruction unit 280 instructs the terminal computer 100 to display the evaluation message and the character image through the network communication unit 210.

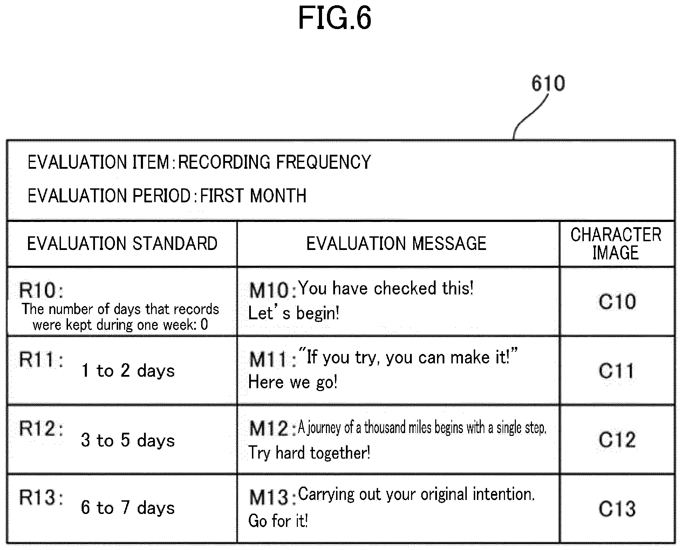

FIG. 6 illustrates exemplary evaluation message data 610 related to the first evaluation item "RECORDING FREQUENCY." The evaluation message data 610 is stored in the evaluation information database unit 270. The evaluation message data 610 is used during an evaluation period of the first month. The evaluation information database unit 270 stores the evaluation message data of the first evaluation item to be used during an evaluation period after the second month.

The evaluation message data 610 includes four evaluation messages M10 to M13 corresponding to four evaluation standards R10 to R13. The evaluation message data 610 is associated with four character images C10 to C13 corresponding to the four evaluation messages M10 to M13. The evaluation selector 260 selects an evaluation message and a character image in accordance with the number of days on which biological information was recorded in the acquired information database unit 230 during the preceding one week. For example, when the number of days on which the information was recorded during the preceding one week is six days, the evaluation selector 260 selects the evaluation message M13 "Carrying out your original intention. Go for it!" corresponding to the evaluation standard R13 "6 to 7 days" and selects the character image C13 corresponding to the evaluation message M13.

In the example of FIG. 6, among the evaluation messages M10 to M13, the evaluation messages M10 and M11 are set as negative evaluations. In the example of FIG. 6, the evaluation standards RIO to R13 are fixed values, but may be variation values set based on at least either the biological information or the behavior information accumulated in the acquired information database unit 230.

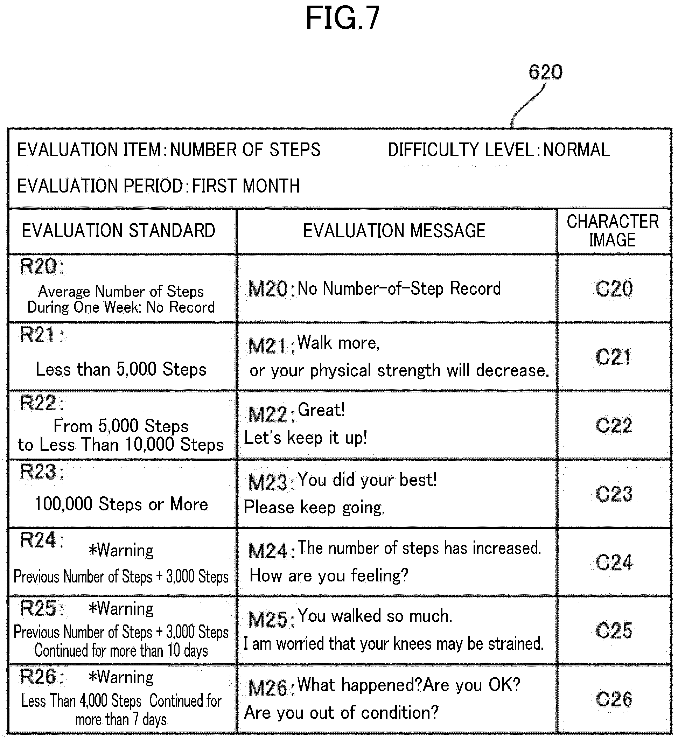

FIG. 7 illustrates exemplary evaluation message data 620 related to the second evaluation item "NUMBER OF STEPS." The evaluation message data 620 is stored in the evaluation information database unit 270. The evaluation message data 620 is used during the evaluation period of the first month. The evaluation information database unit 270 stores the evaluation message data of the second evaluation item to be used during the evaluation period after the second month. The evaluation message data 620 is used if the user selects the difficulty level "NORMAL" from the difficulty levels to be selected by the user in the terminal computer 100. The evaluation information database unit 270 stores the evaluation message data of the second evaluation item to be used at the difficulty levels of "HARD" and "EASY."

The evaluation message data 620 includes four evaluation messages M20 to M23 corresponding to four evaluation standards R20 to R23. The evaluation message data 620 is associated with four character images C20 to C23 corresponding to the four evaluation messages M20 to M23. The evaluation message data 620 includes, in addition to the four evaluation standards R20 to R23, three evaluation messages M24 to M26 corresponding to three types of warning standards R24 to R26 as evaluation standards indicating a warning, and is associated with three character images C24 to C26 corresponding to the three evaluation messages M24 to M26. The evaluation selector 260 preferentially selects the evaluation messages M24 to M26 corresponding to the three types of warning standards R24 to R26 rather than the evaluation messages M20 to M23 corresponding to the four evaluation standards R20 to R23.

The evaluation selector 260 calculates the average number of steps based on the number of steps recorded in the acquired information database unit 230 during the preceding one week, and selects an evaluation message and a character image in accordance with the average number of steps. For example, when the average number of steps during the preceding one week is 6,000, the evaluation selector 260 selects the evaluation message M22 "Great! Let's keep it up!" corresponding to the evaluation standard R22 "From 5,000 Steps to Less Than 10,000 Steps," and selects the character image C22 corresponding to the evaluation message M22. Furthermore, when the average number of steps at this evaluation is greater than the previous number of steps, which is the average number of steps at the previous evaluation, by 3,000 or more and this state is continued for 10 or more days, the evaluation selector 260 selects the evaluation message M25 "You walked so much. I am worried that your knees may be strained" corresponding to the warning standard R25, and selects the character image C25 corresponding to the evaluation message M25.

In the example of FIG. 7, among the evaluation messages M20 to M26, the evaluation messages M20, M21, and R26 are set as negative evaluations. In the example of FIG. 7, the evaluation standards R20 to R26 are fixed values, but may be variation values that increase or decrease based on at least either the biological information or the behavior information accumulated in the acquired information database unit 230.

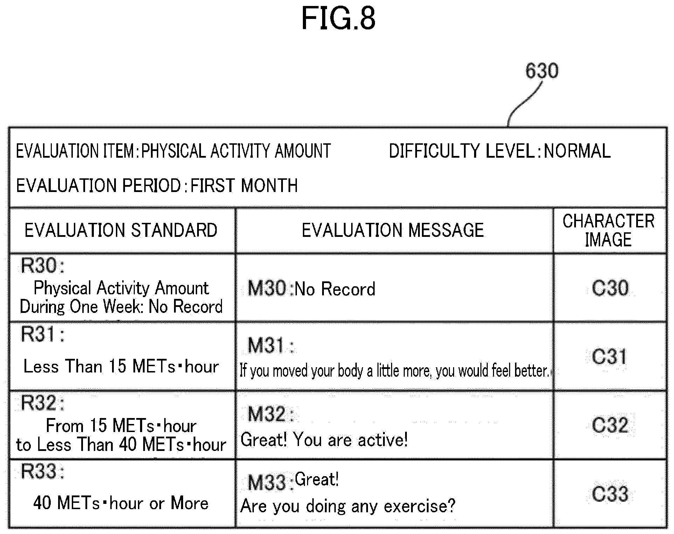

FIG. 8 illustrates exemplary evaluation message data 630 related to the third evaluation item "PHYSICAL ACTIVITY AMOUNT." The evaluation message data 630 is stored in the evaluation information database unit 270. The evaluation message data 630 is used during the evaluation period of the first month. The evaluation information database unit 270 stores the evaluation message data of the third evaluation item to be used during the evaluation period after the second month. The evaluation message data 630 is used if the user selects the difficulty level "NORMAL" from the difficulty levels to be selected by the user in the terminal computer 100. The evaluation information database unit 270 stores the evaluation message data of the third evaluation item to be used at the difficulty levels of "HARD" and "EASY."

The evaluation message data 630 includes four evaluation messages M30 to M33 corresponding to four evaluation standards R30 to R33. The evaluation message data 630 is associated with four character images C30 to C33 corresponding to the four evaluation messages M30 to M33. The evaluation selector 260 calculates a physical activity amount during one week based on metabolic equivalents recorded in the acquired information database unit 230 during the preceding one week, and selects an evaluation message and a character image in accordance with the physical activity amount. For example, when the physical activity amount during the preceding one week is 30 METs·hour, the evaluation selector 260 selects the evaluation message M32 "Great! You are active!" corresponding to the evaluation standard R32 "From 15 METs·hour to Less Than 40 METs·hour," and selects the character image C32 corresponding to the evaluation message M32.

In the example of FIG. 8, among the evaluation messages M30 to M33, the evaluation messages M30 and M31 are set as negative evaluations. In the example of FIG. 8, the evaluation standards R30 to R33 are fixed values, but may be variation values that increase or decrease based on at least either the biological information or the behavior information accumulated in the acquired information database unit 230.

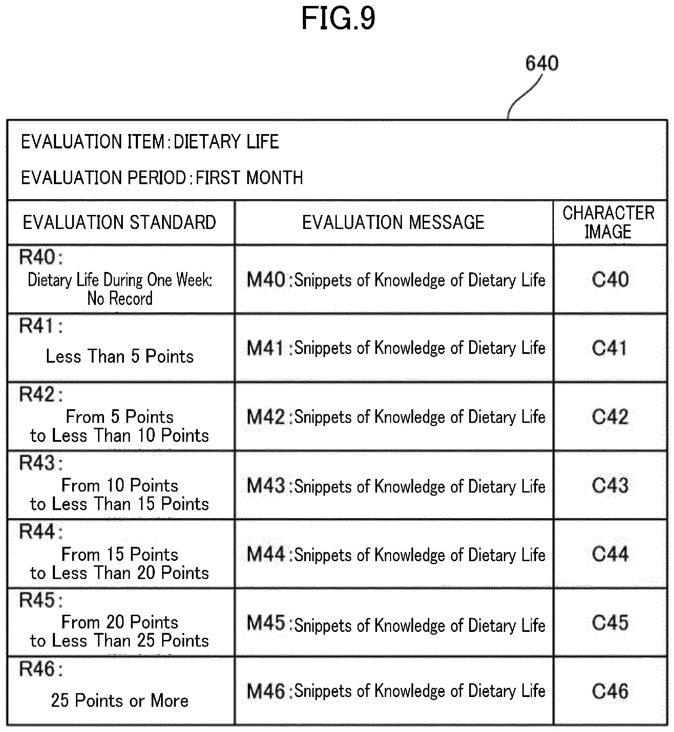

FIG. 9 illustrates exemplary evaluation message data 640 related to the fourth evaluation item "DIETARY LIFE." The evaluation message data 640 is stored in the evaluation information database unit 270. The evaluation message data 640 is used during the evaluation period of the first month. The evaluation information database unit 270 stores the evaluation message data of the fourth evaluation item to be used during the evaluation period after the second month.

The evaluation message data 640 includes seven evaluation messages M40 to M46 corresponding to seven evaluation standards R40 to R46. The evaluation message data 640 is associated with seven character images C40 to C46 corresponding to the seven evaluation messages M40 to M46. The evaluation selector 260 calculates a dietary life value into which a dietary behavior recorded in the acquired information database unit 230 during the preceding one week is converted, and selects an evaluation message and a character image in accordance with the dietary life value. For example, when the dietary life value during the preceding one week is 18 points, the evaluation selector 260 selects snippets of knowledge of dietary life assigned as the evaluation message M44 corresponding to the evaluation standard R44 "From 15 Points to Less Than 20 Points," and selects the character image C44 corresponding to the evaluation message M44.

In the example of FIG. 9, among the evaluation messages M40 to M46, the evaluation messages M40 to M42 are set as negative evaluations. In the example of FIG. 9, the evaluation standards R40 to R46 are fixed values, but may be variation values that increase or decrease based on at least either the biological information or the behavior information accumulated in the acquired information database unit 230.

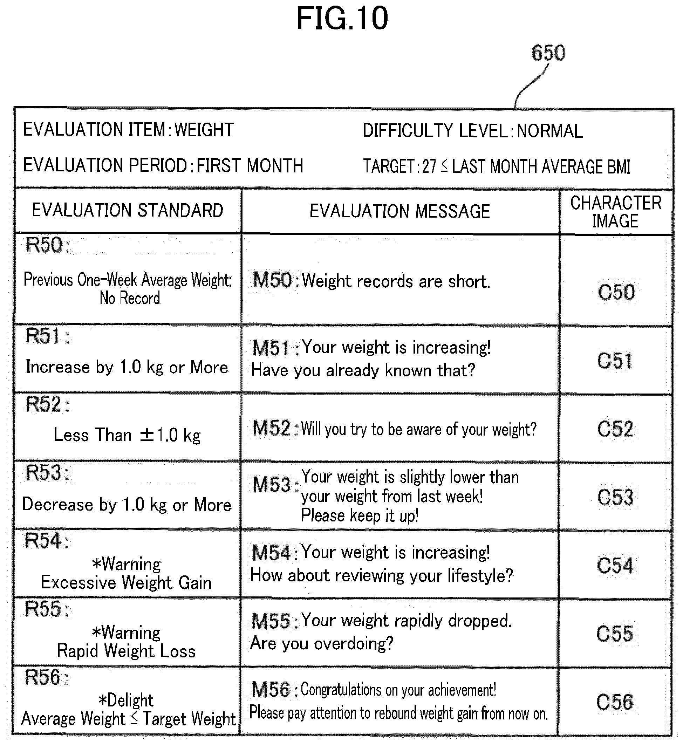

FIG. 10 illustrates exemplary evaluation message data 650 related to the fifth evaluation item "WEIGHT." The evaluation message data 650 is stored in the evaluation information database unit 270. The evaluation message data 650 is used during the evaluation period of the first month. The evaluation information database unit 270 stores the evaluation message data of the fifth evaluation item to be used during the evaluation period after the second month. The evaluation message data 650 is used if the user selects the difficulty level "NORMAL" from the difficulty levels to be selected by the user in the terminal computer 100. The evaluation information database unit 270 stores the evaluation message data of the fifth evaluation item to be used at the difficulty levels of "HARD" and "EASY." The evaluation message data 650 is used for a target person who has an average BMI of 27 or more in the last month. The evaluation information database unit 270 stores the evaluation message data of the fifth evaluation item to be used for other average BMIs.

The evaluation message data 650 includes four evaluation messages M50 to M53 corresponding to four evaluation standards R50 to R53. The evaluation message data 650 is associated with four character images C50 to C53 corresponding to the four evaluation messages M50 to M53. The evaluation message data 650 includes, in addition to the four evaluation standards R50 to R53, two evaluation messages M54 and M55 corresponding to two types of warning standards R54 and R55 as evaluation standards indicating a warning and one evaluation message M56 corresponding to one type of delight standard R56 as an evaluation standard indicating delight, and is associated with three character images C54 to C56 corresponding to these three evaluation messages M54 to M56. The evaluation selector 260 preferentially selects the evaluation messages M54 to M56 corresponding to the two types of warning standards R54 and R55 and the one type of delight standard R56 rather than the evaluation messages M50 to M53 corresponding to the four evaluation standards R50 to R53.

The evaluation selector 260 calculates an average weight based on the weights recorded in the acquired information database unit 230 during the preceding one week, and selects an evaluation message and a character image in accordance with the average weight. For example, when the average weight during the preceding one week is 1.2 kg higher than the average weight at the previous evaluation, the evaluation selector 260 selects the evaluation message M51 "Your weight is increasing! Have you already known that?" corresponding to the evaluation standard R51 "Increase by 1.0 kg or More," and selects the character image C51 corresponding to the evaluation message M51. Furthermore, when the average weight is equal to or lower than a target weight, the evaluation selector 260 selects the evaluation message M56 "Congratulations on your achievement! Please pay attention to rebound weight gain from now on" corresponding to the delight standard R56, and selects the character image C56 corresponding to the evaluation message M56.

In the example of FIG. 10, among the evaluation messages M50 to M56, the evaluation messages M50, M51, and R54 are set as negative evaluations. In the example of FIG. 10, the evaluation standards R50 to R56 are variation values that increase or decrease based on the biological information accumulated in the acquired information database unit 230, but may be variation values that increase or decrease based on the behavior information accumulated in the acquired information database unit 230 or fixed values.

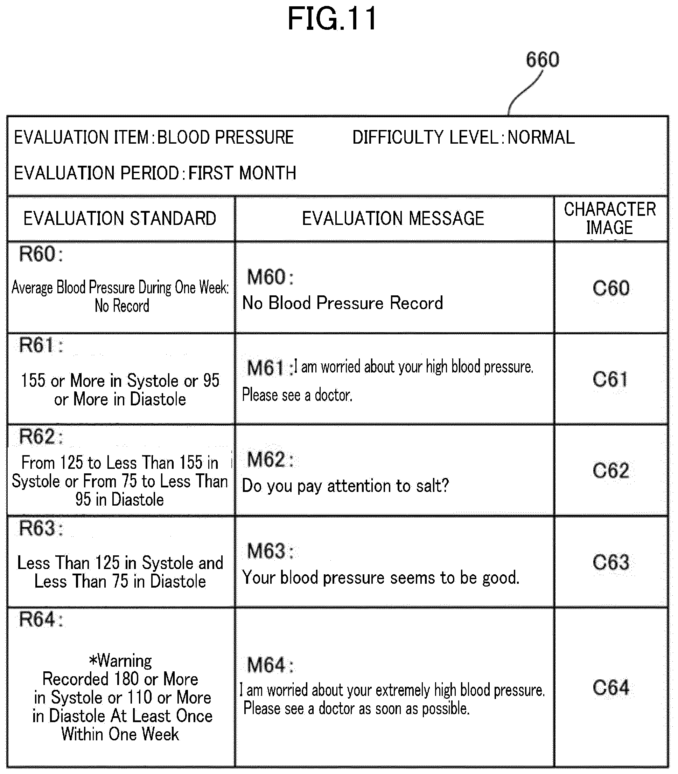

FIG. 11 illustrates exemplary evaluation message data 660 related to the sixth evaluation item "BLOOD PRESSURE." The evaluation message data 660 is stored in the evaluation information database unit 270. The evaluation message data 660 is used during the evaluation period of the first month. The evaluation information database unit 270 stores the evaluation message data of the sixth evaluation item to be used during the evaluation period after the second month. The evaluation message data 660 is used if the user selects the difficulty level "NORMAL" from the difficulty levels to be selected by the user in the terminal computer 100. The evaluation information database unit 270 stores the evaluation message data of the sixth evaluation item to be used at the difficulty levels of "HARD" and "EASY."

The evaluation message data 660 includes four evaluation messages M60 to M63 corresponding to four evaluation standards R60 to R63. The evaluation message data 660 is associated with four character images C60 to C63 corresponding to the four evaluation messages M60 to M63. The evaluation message data 660 includes, in addition to the four evaluation standards R60 to R63, an evaluation message M64 corresponding to a warning standard R64 as an evaluation standard indicating a warning and is associated with a character image C64 corresponding to the evaluation message M64. The evaluation selector 260 preferentially selects the evaluation message M64 corresponding to the warning standard R64 rather than the evaluation messages M60 to M63 corresponding to the four evaluation standards R60 to R63.

The evaluation selector 260 calculates an average blood pressure based on the systolic blood pressures and the diastolic blood pressures recorded in the acquired information database unit 230 during the preceding one week, and selects an evaluation message and a character image in accordance with the average blood pressure. For example, when the average blood pressure during the preceding one week is 118 mmHg in systole and 68 mmHg in diastole, the evaluation selector 260 selects the evaluation message M63 "Your blood pressure seems to be good" corresponding to the evaluation standard R63 "Less Than 125 in Systole and Less Than 75 in Diastole," and selects the character image C63 corresponding to the evaluation message M63. Furthermore, when the average blood pressure was recorded at least once within the preceding one week to be 180 mmHg or more in systole or 110 mmHg or more in diastole, the evaluation selector 260 selects the evaluation message M64 "I am worried about your extremely high blood pressure. Please see a doctor as soon as possible" corresponding to the warning standard R64, and selects the character image C64 corresponding to the evaluation message M64.

In the example of FIG. 11, among the evaluation messages M60 to M64, the evaluation messages M60 to M62, and M64 are set as negative evaluations. In the example of FIG. 11, the evaluation standards R60 to R64 are fixed values, but may be variation values that increase or decrease based on at least either the biological information or the behavior information accumulated in the acquired information database unit 230.

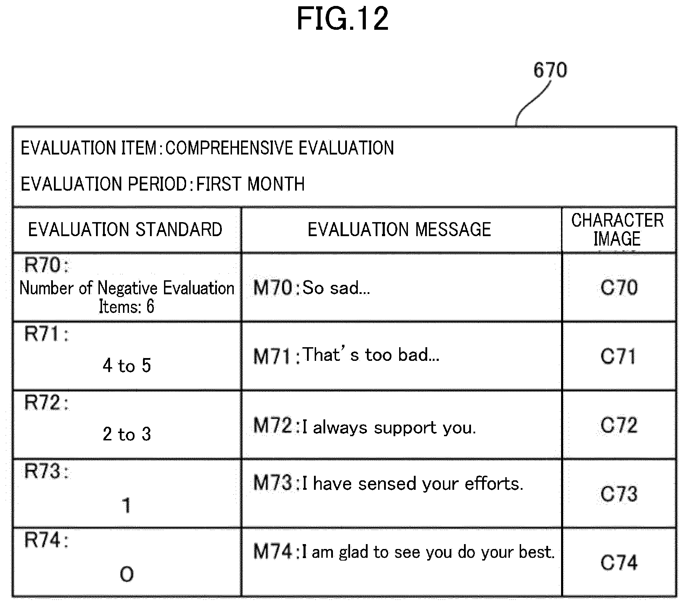

FIG. 12 illustrates exemplary evaluation message data 670 related to the seventh evaluation item "COMPREHENSIVE EVALUATION." The evaluation message data 670 is stored in the evaluation information database unit 270. The evaluation message data 670 is used during the evaluation period of the first month. The evaluation information database unit 270 stores the evaluation message data of the seventh evaluation item to be used during the evaluation period after the second month.

The evaluation message data 670 includes five evaluation messages M70 to M74 corresponding to five evaluation standards R70 to R74. The evaluation message data 670 is associated with five character images C70 to C74 corresponding to the five evaluation messages M70 to M74. The evaluation selector 260 selects an evaluation message and a character image in accordance with the number of negative evaluations for the other evaluation items during the preceding one week. For example, when the number of negative evaluations for the other evaluation items during the preceding one week is three, the evaluation selector 260 selects the evaluation message M72 "I always support you" corresponding to the evaluation standard R72 "2 to 3," and selects the character image C72 corresponding to the evaluation message M72.

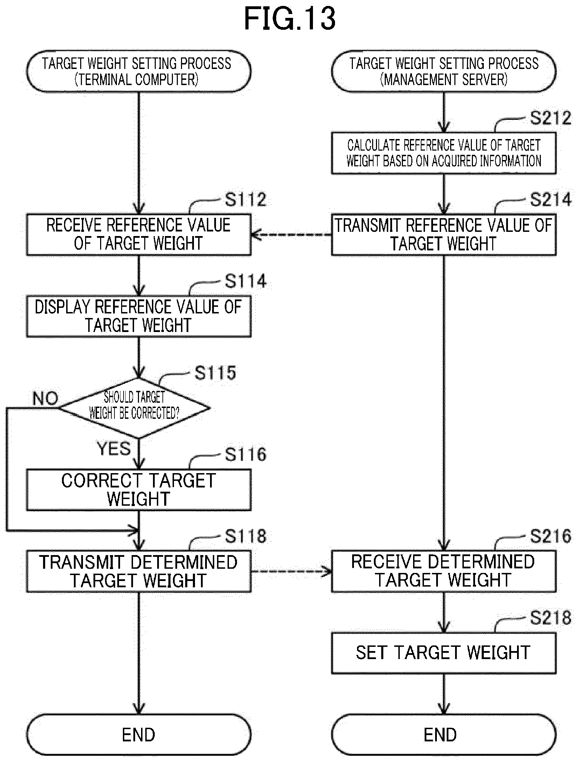

FIG. 13 illustrates operation of the healthcare support system 10. A left portion of the paper of FIG. 13 is a flowchart showing a target weight setting process performed by the terminal computer 100. A right portion of the paper of FIG. 13 is a flowchart showing a target weight setting process performed by the terminal computer 100.

The management server 200 starts the target weight setting process based on an instruction from the terminal computer 100 or the management computer 300. After the target weight setting process is started, the management server 200 calculates a reference value of the target weight based on acquisition information acquired from the terminal computer 100 or the management computer 300 as information indicating the user's weight (step S212). Then, the management server 200 transmits the reference value of the target weight to the terminal computer 100 (step S214). Then, the management server 200 receives a decision value of the target weight from the terminal computer 100 (step S216). Then, the management server 200 sets the decision value received from the terminal computer 100 as the target weight (step S218). Then, the management server 200 ends the target weight setting process.

The terminal computer 100 starts the target weight setting process based on input manipulated by the user or an instruction from the management server 200. After the target weight setting process is started, the terminal computer 100 receives the reference value of the target weight from the management server 200 (step S112). Then, the terminal computer 100 displays the reference value of the target weight on the touch panel 110 (step S114). Then, when the manipulated input for correcting the target weight is received from the user (step S115: "YES"), the terminal computer 100 corrects the target weight based on the input manipulated by the user (step S116) and transmits the corrected value, as the decision value of the target weight, to the management server 200 (step S118). When no manipulated input for correcting the target weight is received from the user (step S115: "NO"), the terminal computer 100 transmits the reference value of the target weight, as the decision value, to the management server 200 (step S118). Then, the terminal computer 100 ends the target weight setting process.

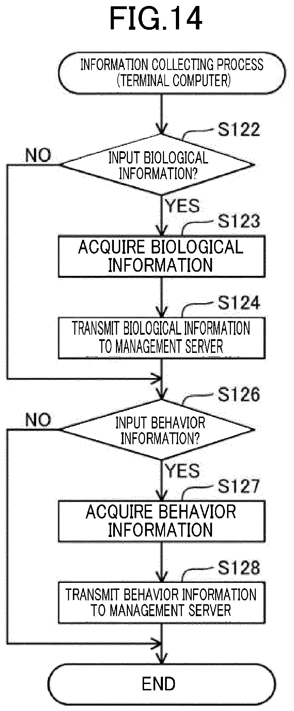

FIG. 14 is a flowchart showing an information collecting process performed by the terminal computer 100. The terminal computer 100 regularly starts the information collecting process. After the information collecting process is started, the terminal computer 100 determines whether it has received biological information from the biological information detection device 80 via the short-range wireless communication (step S122). When the biological information has been received from the biological information detection device 80 (step S122: "YES"), the terminal computer 100 operates as the biological information acquisition unit 152 to acquire the biological information from the biological information detection device 80 via the short-range wireless communication (step S123). Then, the terminal computer 100 operates as the terminal information transmitter 156 to transmit the biological information acquired from the biological information detection device 80 to the management server 200 (step S124).

When no biological information has been received from the biological information detection device 80 (step S122: "NO") or after the biological information acquired from the biological information detection device 80 has been transmitted to the management server 200 (step S124), the terminal computer 100 determines whether it has received behavior information based on input manipulated by the user (step S126). When the behavior information has been received based on the input manipulated by the user (step S126: "YES"), the terminal computer 100 operates as the behavior information acquisition unit 154 to acquire the behavior information based on the input manipulated by the user (step S127, see the screen 510 of FIG. 3). Then, the terminal computer 100 operates as the terminal information transmitter 156 to transmit the behavior information acquired based on the input manipulated by the user to the management server 200 (step S128). When no behavior information has been received based on the input manipulated by the user (step S126: "NO") or after the behavior information acquired based on the input manipulated by the user has been transmitted to the management server 200 (step S128), the terminal computer 100 ends the information collecting process.

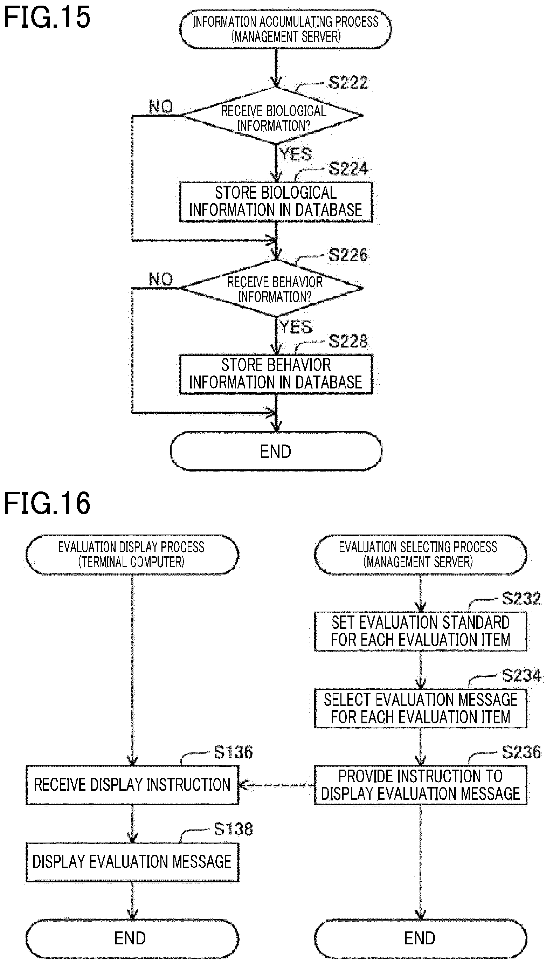

FIG. 15 is a flowchart showing an information accumulating process performed by the management server 200. The management server 200 starts the information accumulating process on a regular basis (for example, once a day). After the information accumulating process is started, the management server 200 determines whether it has received the biological information from the terminal computer 100 (step S222). When the biological information has been received from the terminal computer 100 (step S222: "YES"), the management server 200 operates as the information acquisition unit 220 to store the biological information from the terminal computer 100 in the acquired information database unit 230 (step S224).

When the biological information has not been received from the terminal computer 100 (step S222: "No") or after the biological information has been stored in the acquired information database unit 230 (step S224), the management server 200 determines whether it has received behavior information from the terminal computer 100 (step S226). When the behavior information has been received from the terminal computer 100 (step S226: "YES"), the management server 200 operates as the information acquisition unit 220 to store the behavior information from the terminal computer 100 in the acquired information database unit 230 (step S228). When the behavior information has not been received from the terminal computer 100 (step S226: "No") or after the behavior information has been stored in the acquired information database unit 230 (step S228), the management server 200 ends the information accumulating process.

FIG. 16 illustrates operation of the healthcare support system 10. A left portion of the paper of FIG. 16 is a flowchart showing an evaluation display performed by the terminal computer 100. A right portion of the paper of FIG. 16 is a flowchart showing an evaluation selecting process performed by the terminal computer 100.

The management server 200 starts the evaluation selecting process on a regular basis (for example, twice a week). After the evaluation selecting process is started, the management server 200 operates as the evaluation standard setting unit 240 to set an evaluation standard for each of a plurality of management items, and stores the evaluation standard in the evaluation standard storage unit 250 (step S232).

After setting the evaluation standard for each of the plurality of management items (step S232), the management server 200 operates as the evaluation selector 260 to select an evaluation message for each of the plurality of management items based on the biological information and the behavior information acquired during the predetermined period and accumulated in the acquired information database unit 230 and the evaluation standard for each of the plurality of management items stored in the evaluation standard storage unit 250 (step S234). The management server 200 selects a character image corresponding to the evaluation message for each of the plurality of management items, in addition to the evaluation message for each of the plurality of management items.

After selecting the evaluation message for each of the plurality of management items (step S234), the management server 200 operates as the display instruction unit 280 to instruct the terminal computer 100 to display the evaluation message for each of the plurality of management items such that the user can recognize the evaluation message for each of the plurality of management items (step S236). The management server 200 instructs the terminal computer 100 to display the character image for each of the plurality of management items, in addition to the evaluation message for each of the plurality of management items. After instructing the terminal computer 100 to display the evaluation message (step S236), the management server 200 ends the evaluation selecting process.

The terminal computer 100 starts the evaluation display process based on an instruction from the management server 200. After the evaluation display process is started, the terminal computer 100 receives, from the management server 200, an instruction for displaying the evaluation message for each of the plurality of management items (step S136). The terminal computer 100 receives, from the management server 200, an instruction for displaying the character image for each of the plurality of management items, in addition to the instruction for displaying the evaluation message for each of the plurality of management items.

After receiving the instruction for displaying the evaluation message for each of the plurality of management items from the management server 200 (step S136), the terminal computer 100 operates as the evaluation display 158 to display the evaluation message for each of the plurality of management items on the touch panel 110 such that the user can recognize the evaluation message for each of the plurality of management items (step S138, see the screen 520 of FIG. 4). The terminal computer 100 displays the character image for each of the plurality of management items on the touch panel 110 in addition to the evaluation message for each of the plurality of management items. After displaying the evaluation message for each of the plurality of management items (step S138), the terminal computer 100 ends the evaluation display process.

According to the embodiment described above, based on the biological information and the behavior information acquired during the predetermined period, the management server 200 selects the evaluation message for evaluating the user's healthcare efforts for each of the plurality of management items to allow the user to recognize these evaluation messages in the terminal computer 100. Consequently, it is possible to implement health guidance according to the user's situation while reducing the burden on a healthcare leader. Furthermore, evaluating the user's healthcare efforts using the character image in addition to the evaluation message can improve the user's eagerness to make healthcare efforts.

Furthermore, the user's healthcare efforts can be evaluated based on the biological information detectable by at least one of a body composition meter, a blood pressure meter, and an activity meter. Furthermore, the user's healthcare efforts can be evaluated based on the behavior information indicating the user's dietary behavior.

Furthermore, since the plurality of management items include the first to seventh management items, it is possible to evaluate efforts for prevention and treatment of type II diabetes as the user's healthcare efforts.

Furthermore, the evaluation standard setting unit 240 is capable of setting an evaluation standard related to at least one of the plurality of management items based on at least either the biological information or the behavior information accumulated in the acquired information database unit 230. Thus, the user's past performance for healthcare can be reflected in the evaluation standard.

The present invention is not limited to the foregoing embodiments, examples, and variations, but may be embodied in various other forms without departing from the spirit of the present invention. For example, in order to solve, partially or entirely, the above-mentioned problem or yield, partially or entirely, the above-mentioned advantages, technical features of the embodiments, examples, and variations corresponding to technical features of the modes described in the section "SUMMARY OF THE INVENTION" can be replaced or combined as appropriate. Also, the technical feature(s) may be eliminated as appropriate unless the present specification mentions that the technical feature(s) is mandatory.

The number of the plurality of management items merely needs to be two or more, and may be six or less or eight or more. The evaluation standard for the management item may be a fixed value, a variation value set based on the biological information, a variation value set based on the behavior information, or a variation value set based on both the biological information and the behavior information. For example, when the behavior information indicating the dietary life implies excessive intake, the evaluation standard setting unit 240 of the management server 200 may set an evaluation standard value of at least one of the management items, the "NUMBER OF STEPS," the "PHYSICAL ACTIVITY AMOUNT," or the "WEIGHT" to be greater than a standard value. Furthermore, when the at least one of the management items, i.e., the "NUMBER OF STEPS," the "PHYSICAL ACTIVITY AMOUNT," or the "WEIGHT" sufficiently satisfies its standard value, the evaluation standard setting unit 240 of the management server 200 may set an evaluation standard value of the management item "DIETARY LIFE" to be less than the standard value.

### DESCRIPTION OF REFERENCE CHARACTERS

- 10: Healthcare Support System
- 80: Biological Information Detection Device
- 100: Terminal Computer
- 110: Touch Panel
- 120: Network Communication Unit
- 130: Short-Range Wireless Communication Unit
- 150: Health Support Application
- 152: Biological Information Acquisition Unit
- 154: Behavior Information Acquisition Unit
- 156: Terminal Information Transmitter
- 158: Evaluation Display
- 200: Management Server
- 210: Network Communication Unit
- 220: Information Acquisition Unit
- 230: Acquired Information Database Unit
- 240: Evaluation Standard Setting Unit
- 250: Evaluation Standard Storage Unit
- 260: Evaluation Selector
- 270: Evaluation Information Database Unit
- 280: Display Instruction Unit
- 300: Management Computer
- 502: Title Bar
- 508: Tab
- 510: Screen
- 512: Date Input Field
- 514: Input Button
- 516: Send Button
- 520: Screen
- 521a: Display Section
- 521b: Display Section
- 521c: Display Section
- 522a: Display Section
- 522b: Display Section
- 522c: Display Section
- 523a: Display Section
- 523b: Display Section
- 523c: Display Section
- 524a: Display Section
- 524b: Display Section
- 524c: Display Section
- 525a: Display Section
- 525b: Display Section
- 525c: Display Section
- 526a: Display Section
- 526b: Display Section
- 526c: Display Section
- 527a: Display Section
- 527b: Display Section
- 527c: Display Section
- 571a: Display Section

## Claims

1. A healthcare support system that supports healthcare for a user, the healthcare support system comprising:
a terminal computer operable by the user; and
a management server capable of exchanging data with the terminal computer via a computer network, wherein
the terminal computer includes:
a biological information acquisition unit configured to acquire biological information of the user from a biological information detection device via short-range wireless communication, the biological information detection device detecting the biological information;
a behavior information acquisition unit configured to acquire behavior information indicating a behavior of the user based on input manipulated by the user; and
a terminal information transmitter configured to transmit the biological information acquired by the biological information acquisition unit and the behavior information acquired by the behavior information acquisition unit to the management server,
the management server includes:
an acquired information database unit configured to accumulate the biological information and the behavior information transmitted from the terminal computer;
an evaluation standard setting unit configured to set an evaluation standard for each of a plurality of management items related to the healthcare of the user;
an evaluation selector configured to select an evaluation message for evaluating efforts for the healthcare of the user during a predetermined period for each of the management items based on the biological information and the behavior information acquired during the predetermined period and accumulated in the acquired information database unit and the evaluation standard for each of the management items; and
a display instruction unit configured to instruct the terminal computer to display the evaluation message for each of the management items such that the user is able to recognize the evaluation message for each of the management items, and
the evaluation standard setting unit of the management server sets an evaluation standard related to at least one of the management items based on at least either the biological information or the behavior information accumulated in the acquired information database unit.

2. The healthcare support system of claim 1, wherein
based on the biological information and the behavior information acquired during the predetermined period and accumulated in the acquired information database unit and the evaluation standard for each of the management items, the evaluation selector of the management server selects the evaluation message for each of the management items, and selects a character image corresponding to the evaluation message for each of the management items, and
the display instruction unit of the management server instructs the terminal computer to display the evaluation message and the character image for each of the management items such that the user is able to recognize the evaluation message and the character image for each of the management items.

3. The healthcare support system of claim 1 or 2, wherein
the biological information detection device, which provides the biological information acquisition unit with the biological information, includes at least one of a body composition meter, a blood pressure meter, and an activity meter.

4. The healthcare support system of any one of claims 1 to 3, wherein
the behavior information acquired by the behavior information acquisition unit includes information indicating a dietary behavior of the user.

5. The healthcare support system of any one of claims 1 to 4, wherein
the management items comprise at least two of:
a first management item to manage a recording frequency with which the biological information and the behavior information have been recorded;
a second management item to manage the number of steps of the user;
a third management item to manage a physical activity amount of the user;
a fourth management item to manage dietary life of the user;
a fifth management item to manage a weight of the user;
a sixth management item to manage a blood pressure of the user; and
a seventh management item to comprehensively manage the other ones of the management items.

6. The healthcare support system of any one of claims 1 to 4, wherein
the evaluation standard setting unit of the management server sets the evaluation standard related to at least one of the management items based on at least either biological information or behavior information different from biological information and behavior information evaluated in the at least one of the management items, out of the biological information and the behavior information accumulated in the acquired information database unit.

7. A management server capable of exchanging data with a terminal computer operable by a user via a computer network in a healthcare support system that supports healthcare for the user, the management server comprising:
an information acquisition unit configured to acquire biological information of the user and behavior information indicating a behavior of the user from the terminal computer via the computer network, the biological information being acquired by the terminal computer from a biological information detection device via short-range wireless communication, the biological information detection device detecting the biological information, the behavior information being acquired by the terminal computer based on input manipulated by the user;
an acquired information database unit configured to accumulate the biological information and the behavior information acquired by the information acquisition unit;
an evaluation standard setting unit configured to set an evaluation standard for each of a plurality of management items related to the healthcare of the user;
an evaluation selector configured to select an evaluation message for evaluating efforts for the healthcare of the user during a predetermined period for each of the management items based on the biological information and the behavior information acquired during the predetermined period and accumulated in the acquired information database unit and the evaluation standard for each of the management items; and
a display instruction unit configured to instruct the terminal computer to display the evaluation message for each of the management items such that the user is able to recognize the evaluation message for each of the management items, wherein
the evaluation standard setting unit sets an evaluation standard related to at least one of the management items based on at least either the biological information or the behavior information accumulated in the acquired information database unit.
